(19) Europäisches Patentamt
European Patent Office
Office européen des brevets

(11) Veröffentlichungsnummer: **0 003 053**
**B1**

(12) **EUROPÄISCHE PATENTSCHRIFT**

(21) Anmeldenummer: **78101859.3**

(22) Anmeldetag: **28.12.78**

(51) Int. Cl.³: **C 07 C 99/00,**
C 07 C 101/54

# (54) Verfahren zur kontinuierlichen Herstellung von Anthranilsäure

(30) Priorität: **11.01.78 DE 2800963**

(43) Veröffentlichungstag der Anmeldung:
**25.07.79 Patentblatt 79/15**

(45) Bekanntmachung des Hinweises auf die Patenterteilung:
**26.11.80 Patentblatt 80/24**

(84) Benannte Vertragsstaaten:
**BE CH DE FR GB NL**

(56) Entgegenhaltungen:
**FR - A - 2 065 044**
**GB - A - 1 436 810**
**US - A - 2 878 281**

(73) Patentinhaber: **BASF Aktiengesellschaft**
**Carl-Bosch-Strasse 38**
**D - 6700 Ludwigshafen (DE)**

(72) Erfinder: **Kilpper, Gerhard, Dr.**
**Waldstrasse 3**
**D - 6719 Battenberg (DE)**
**Grimmer, Johannes**
**Duererstrasse 12**
**D - 6700 Ludwigshafen (DE)**

**EP 0 003 053 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur kontinuierlichen Herstellung von Anthranilsäure

Die Erfindung betrifft ein Verfahren zur kontinuierlichen Herstellung von Anthranilsäure durch zweistufige Umsetzung von phthalamidsaurem und/oder phthalimidsaurem Alkali mit Alkalihypohalogeniten, indem die erste Stufe weitgehend adiabatisch und beide Stufen unter Verwendung hoher Strömungsgeschwindigkeiten und unterschiedlicher Temperaturen durchgeführt werden, wobei zunächst Phthalimid und/oder Phthalamidsäure in einer bestimmten, überschüssigen Alkalilaugenmenge gelöst werden und erst dann ohne weiteren Zusatz von überschüssigem Alkali die Lösung mit Hypohalogeniten vermischt und umgesetzt wird.

Es ist aus der deutschen Patentschrift 1 224 748 bekannt, daß man phthalamidsaures Alkali durch Oxidation mit Alkalihypochlorit kontinuierlich zur Anthranilsäure umsetzt. Die Ausgangsstoffe werden in Form ihrer gekühlten, wäßrigen Lösungen miteinander in einer gekühlten Mischkammer vermischt und in der ersten Reaktionsstufe, der Bildung von Phenylisocyanat-2-carbonsäure, in dem ein Kühlsystem enthaltenden Teil einer Reaktionskolonne umgesetzt. In der zweiten Stufe, der Bildung von Anthranilsäure, soll die Reaktionstemperatur 70°C nicht übersteigen. In der Beschreibung wird auf die Wichtigkeit, insbesondere in der ersten Stufe die Reaktionswärme durch Kühlung abzuführen, hingewiesen und eine Maximaltemperatur von +10°C für die Bildung der Phenylisocyanat-2-carbonsäure genannt. Auch bei diskontinuierlicher Verfahrensweise der Umsetzung wurde bisher auf gute Kühlung Wert gelegt.

Aus den deutschen Auslegeschriften 1 950 281 und 2 000 698 ist ein Verfahren zur kontinuierlichen Herstellung von Anthranilsäure durch Umsetzung von phthalamidsaurem und/oder phthalimidsaurem Alkali mit Hypohalogeniten in wäßrigem Medium bekannt, wobei man

a) ein wäßrige Lösung von phthalamidsaurem und/oder phthalimidsaurem Alkali und eine wäßrige Lösung von Alkalihypochlorit in einer Mischvorrichtung mischt,
b) die erhaltene Mischung im ersten Teil eines engen Reaktionsrohes mit hoher Strömungsgeschwindigkeit bei 10 bis 50°C unter weitgehend adiabatischen Bedingungen umsetzt, anschließend
c) die aus dem ersten Teil des Reaktionsrohres mit hoher Strömungsgeschwindigkeit abströmende Umsetzungsmischung im zweiten Teil des genannten Rohres bei 60 bis 80°C zu Ende umsetzt und
d) aus der abströmenden alkalischen Umsetzungsmischung in üblicher Weise Anthanilsäure und/oder Isatosäureanhydrid abtrennt, und gegebenenfalls während der Stufe b) und/oder der Stufe c) ein Reduktionsmittel zusetzt.

Bei diesem Verfahren wird nur ein Teil des freien Alkalihydroxids schon beim Lösen des Ausgangsstoffes verwendet, ein weiterer Teil der Hypochloritlösung zugesetzt. Vorteilhaft gelangen wäßrige Lösungen von 10 bis 50 Gewichtsprozent Phthalimid und/oder Phthalamid zur Anwendung, die von 1 bis 1,1 Mol Alkalihydroxid je Mol Phthalimid/Phthalamid enthalten. Es wird angegeben, daß die wäßrigen Hypohalogenitlösungen vorteilhaft von 8 bis 15 Gewichtsprozent Hypohalogenit und von 0 bis 3, vorzugsweise von 0,02 bis 2,1 Mol Alkalihydroxid je Mol Phthalimid/Phthalamidsäure enthalten. In Beispiel 1 (Herstellung von Anthranilsäure) befindet sich Alkalihydroxid sowohl in der Phthalimidlösung in einer Menge von 1,1 Mol NaOH je Mol Phthalimid als auch in der Hypochloritlösung (1,4 Mol NaOH, bezogen auf 1 Mol Phthalimid). Die Auslegeschrift 1 950 281 gibt an, daß durch Einstellung der Ausgangslösungen in Bezug auf Alkalikonzentration die Bildung des Endstoffs beeinflußt wird. Bei einer Menge von 0,9 bis 1,1 Mol Alkali je Mol Phthalimid bzw. Phthalamidsäure im Ausgangsgemisch erhält man Isatosäureanhydrid. Nur für diesen Fall sieht die Auslegeschrift, wie Beispiel 2 zeigt, eine Zugabe des gesamten Alkali zum Ausgangsstoff vor.

Die deutsche Offenlegungsschrift 2 328 757 beschreibt ein Verfahren zur Herstellung von Aminen, z.B. auch Anthranilsäure, durch Umsetzung von Carbonsäureamiden mit Hypochloriten in Gegenwart von Brom, Jod und/oder Halogenamiden und überschüssigem Alkalihydroxid. Es wird angegeben, daß vorteilhaft wäßrige Suspensionen von 1 bis 50 Gewichtsprozent Ausgangscarbonsäureamid zur Anwendung gelangen. Die wäßrigen Hypochloritlösungen enthalten im allgemeinen von 5 bis 15, vorzugsweise von 12 bis 14 Gewichtsprozent Hypochlorit und können zusätzlich von 0,2 bis 2,5 Mol, vorzugsweise 1 bis 2,1 Mol Alkalihydroxid je Mol Hypochlorit enthalten. Im Ausgangsgemisch beider Ausgangsstoffe kommen im allgemeinen Mengen von insgesamt 0,2 bis 2,5 Mol, vorzugsweise 1 bis 2,1 Mol Alkalihydroxid (nicht eingerechnet das im Hypochlorit enthaltene Alkali), bezogen auf 1 Mol Ausgangscarbonsäureamid und Carbonamidgruppe am Molekül, in Frage. Enthält die wäßrige Hypochloritlösung kein freies Alkalihydroxid, so werden am Anfang oder im Laufe der Umsetzung zweckmäßig von 0,2 bis 2, vorzugsweise 1 bis 2 Mol Alkalihydroxid pro Mol Hypochlorit zugeführt. Es wird beschrieben, daß für die Reaktion einem Gemisch von Ausgangscarbonsäureamid, Katalysator und Wasser eine wäßrige Lösung des Hypohalogenits zugeführt und das Gemisch während 1 bis 4 000 Sekunden bei der Reaktionstemperatur gehalten wird. Dann wird im Falle von Phthalamidsäure wäßrige Alkalilauge zugeführt und das Gemisch eine Sekunde bis zu 3 Stunden bei der Reaktionstemperatur, gegebenenfalls unter Erwärmen gehalten. Je höher man die Reaktionstemperatur wählt, dest kürzer hält man zweckmäßig die Reaktionszeit bis zur Zugabe der Alkalilauge. In einer bevorzugten Ausführungsform wird das Ausgangscarbonsäureamid, z.B. Phthalamidsäure, zuerst aus Carbonsäureanhydrid, Ammoniak und gegebenenfalls Alkalihydroxid bei einer Temperatur von in der Regel 20 bis 80°C hergestellt und das so gebildete Reaktionsgemisch ohne Isolierung des Reaktionsproduktes direkt als Ausgangsstoff nach dem erfindungsgemäßen Verfahren umgesetzt. In Beispiel 1 wird so zuerst phthalamidsaures Salz hergestellt, wobei sich in der Lösung kein überschüssiges Natriumhydroxid je Mol Phthalamidsäure befinden, diese Lösung dann mit Chlorlauge, die kein überschüssiges Alkalihydroxid enthält, vermischt und schließlich 2 Mol Natriumhydroxid je Mol Phthalamidsäure zugesetzt.

Ein weiteres in der deutschen Offenlegungsschrift 2 357 749 beschriebenes Verfahren zeigt die entsprechende Herstellung von Aminen durch Umsetzung von Carbonsäureamiden mit Hypochloriten in Gegenwart von überschüssigem Alkalihydroxid und in Gegenwart von Polymerisationsinhibitoren. Vorteilhaft gelangen wäßrige Suspensionen von 1 bis 50 Gewichtsprozent Ausgangscarbonsäureamid zur Anwendung. Die wäßrigen Hypochloritlösungen enthalten auch hier im allgemeinen von 5 bis 15, vorzugsweise von 12 bis 14 Gewichtsprozent Hypochlorit und können zusätzlich von 0,2 bis 2,5 Mol, vorzugsweise 1 bis 2.1 Mol, Alkalihydroxid je Mol Hypochlorit enthalten. Im Ausgangsgemisch beider Ausgangsstoffe kommen im allgemeinen Mengen von insgesamt 0,2 bis 2,5 Mol, vorzugsweise 1 bis 2,1 Mol Alkalihydroxid (nicht eingerechnet das im Hypochlorit enthaltene Alkali), bezogen auf 1 Mol Ausgangscarbonsäureamid und Carbonamidgruppe am Molekül, in Frage. Enthält die wäßrige Hypochloritlösung kein freies Alkalihydroxid, so werden am Anfang oder im Laufe der Umsetzung zweckmäßig von 0,2 bis 2, vorzugsweise 1 bis 2 Mol Alkalihydroxid pro Mol Hypochlorit zugeführt. Die Arbeitsweise bezüglich Zugabe von Alkalilauge entspricht der deutschen Offlengungsschrift 2 328 757, wie auch alle Beispiele zeigen. In beiden Offenlegungsschriften wird ausdrücklich angegeben, daß man nur bei anderen Carbonsäureamides, nicht bei Phthalamidosäure, zweckmäßig das Alkali von Anfang an mit dem Ausgangsgemisch vereint und während einer Sekunde bis zu 3 Stunden die Umsetzung durchführt.

Es wurde nun gefunden, daß man Anthranilsäure durch Umsetzung von phthalamidsaurem und/oder phthalimidsaurem Alkali mit Hypohalogeniten in wäßrigem Medium vorteilhaft herstellt, wenn man

a) Phthalimid und/oder Phthalamidsäure in wäßriger Alkalilauge mit einem Verhältnis von 3 bis 3,5 Mol Alkalihydroxid je Mol Phthalimid und/oder von 2 bis 2,5 Mol Alkalihydroxid je Mol Phthalamidsäure löst,
b) die so gebildete wäßrige Lösung von phthalamidsaurem und/oder phthalimidsaurem Alkali und eine wäßrige Lösung von Alkalihypochlorit in einer Mischvorrichtung mischt,
c) die erhaltene Mischung im ersten Teil eines Reaktionsrohres mit hoher Strömungsgeschwindigkeit bei 10 bis 54°C unter weitgehend adiabatischen Bedingungen umsetzt, anschließend,
d) die aus dem ersten Teil des Reaktionsrohres mit hoher Strömungsgeschwindigkeit abströnende Umsetzungsmischung im zweiten Teil des genannten Rohres bei 55 bis 90°C zu Ende umsetzt und
e) aus der abströmenden alkalischen Umsetzungsmischung in üblicher Weise Anthranilsäure abtrennt.

Weiterhin wurde gefunden, daß man das Verfahren vorteilhaft ausführt, wenn man dem Reaktionsgemisch während der Stufe c) oder d) ein Reduktionsmittel zusetzt.

Weiterhin wurde gefunden, daß man das Verfahren vorteilhaft ausführt, wenn die Umsetzung in Gegenwart von Brom, Jod und/oder Halogenamiden der Formel

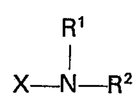

I,

worin $R^1$ eine Sulfonsäuregruppe, einen Sulfonatrest, eine Sulfonamidgruppe bezeichnet, $R^2$ ein Wasserstoffatom, einen aliphatischen Rest, ein Chloratom oder Bromatom bedeutet, X ein Chloratom, Bromatom oder Wasserstoffatom bezeichnet, $R^1$ und $R^2$ darüber hinaus auch zusammen mit dem benachbarten Stickstoffatom Glieder eines heterocyclischen Restes, der mindestens eine dem Stickstoffatom benachbarte Sulfongruppe oder Phosphonylgruppe der Formel

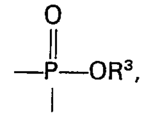

worin $R^3$ für ein Wasserstoffatom oder ein Alkaliatom steht, enthält, bezeichnen können, oder $R^1$ und $R^2$ zusammen auch den Rest

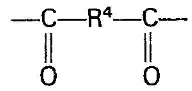

bedeuten können, worin $R^4$ für einen Alkylenrest, den Rest

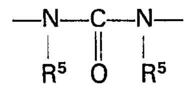

oder den Rest

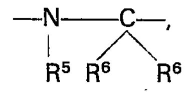

$R^5$ für ein Wasserstoffatom, ein Chloratom oder Bromatom und $R^6$ für einen aliphatischen Rest stehen, durchgeführt wird.

Die Umsetzung läßt sich für den Fall der Verwendung von Natriumhydroxid und Natriumhypochlorit durch die folgenden Formeln wiedergeben:

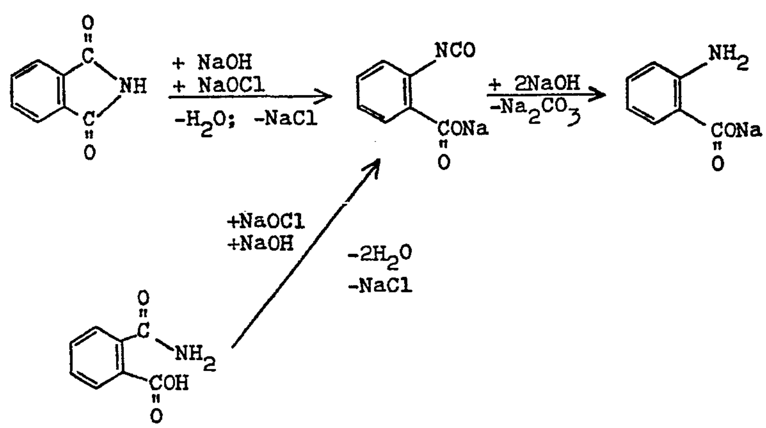


Im Vergleich zu den bekannten Verfahren liefert das Verfahren nach der Erfindung Anthranilsäure auf einfacherem und wirtschaftlicherem Wege in teilweise besserer Ausbeute und Reinheit und wesentlich besserer Raum-Zeit-Ausbeute. Die Reaktion verläuft schneller und kann somit in wesentlich kleineren Rohrreaktoren durchgeführt werden. Der Endstoff fällt in einer gröberen und besser ausgebildeten Kristallform von geringerer Restfeuchte an. Man erhält weniger schmierige, besser filtrierbare und trockenbare Kristalle, die im nachfolgenden Feststofftransport der Trocknungsanlage einen schnelleren und störungsfreieren Betrieb erlauben. Außerdem ist der Zusatz der gesamten überschüssigen Alkalilauge in Stufe a) vorteilhaft. Denn bei den bekannten Verfahren werden die für den Ablauf des chemischen Prozesses benötigte Lauge nur zum Teil der Phthalimid (Phthalamid)-lösung, zum Teil der Bleichlauge oder erst nachträglich dem Gesamtgemisch zudosiert. Beim Betrieb ergeben sich durch Regelschwankungen Ungenauigkeiten in der Laugedosierung, die bei den bekannten Verfahren den Prozeßablauf negativ beeinflussen, z.B. besteht bei Unterschuß von Natronlauge in der Phthalimidlösung die Gefahr, daß Phthalimid ungelöst bleibt und Verstopfungen hervorruft. Schwankungen der Natronlaugemenge in der Bleichlauge führen zu Konzentrationsänderungen, die die Ausbeute beeinflussen. Mit einer zusätzlichen Dosierung von Lauge in das Gesamtgemisch der Ausgangsstoffe (Phthalimid bzw. Phthalamidsäure und Hypochlorit) wird die Anzahl der erforderlichen Regelungen noch weiter erhöht. Entsprechend ist der Betrieb des erfindungsgemäßen Verfahrens vergleichsweise sicherer und erfordert weniger Betriebs- und Überwachungspersonal. Die Gesamtbetriebszeit, einschließlich Herstellung des wäßrigen Ausgangsgemisches und Aufarbeitung des Reaktionsgemisches, ist bei dem erfindungsgemäßen Verfahren kürzer. Alle diese vorteilhaften Ergebnisse sind im Hinblick auf den Stand der Technik überraschend.

Der Ausgangsstoff wird in Stufe a) in wäßriger Alkalilauge, zweckmäßig Kalilauge und insbesondere Natronlauge, gelöst. Vorteilhaft gelangen wäßrige Lösungen von 10 bis 50, bevorzugt 15 bis 30 Gewichtsprozent (bezogen auf die reine Wassermenge) Phthalimid und/oder Phthalamidsäure zur Anwendung, die von 3 bis 3,5, bevorzugt 3,1 bis 3,2 Mol Alaklihydroxid je Mol Phthalimid und/oder 2 bis 2,5, bevorzugt 2,1 bis 2,2 Mol Alkalihydroxid je Mol Phthalamidsäure enthalten. Die Lösung wird zweckmäßig bei einer Temperatur von —5 bis +50°C, vorzugsweise von 20 bis 30°C, drucklos oder

unter Druck, kontinuierlich durchgeführt. Verwendet man Katalysatoren, z.B. die in der deutschen Offenlegungsschrift 2 357 749 oder vorteilhaft die in der deutschen Offenlegungsschrift 2 328 757 beschriebenen Katalysatoren, so werden sie zweckmäßig schon in Stufe a) dem Ausgangsstoff bzw. seiner Lösung zugesetzt. Man kann aber den Katalysator, zweckmäßig im Gemisch mit Wasser, auch dem Ausgangsgemisch getrennt oder zusammen mit dem Hypohalogenit zusetzen.

Als Katalysatoren kommen vorteilhaft Brom, Jod und/oder die vorgenannten Halogenamide I, im allgemeinen in einer Menge von 0,0001 bis 0,1, vorzugsweise von 0,001 bis 0,01 Mol Katalysator je Mol Phthalimid bzw. Phthalamidsäure in Betracht. Anstelle der genannten Stoffe können auch Verbindungen, die unter den Reaktionsbedingungen solche Stoffe bilden, verwendet werden, z.B. Bromide und Jodide anstelle von Brom oder Jod. Zweckmäßig wählt man wasserlösliche Halogenide. Diese Halogenide kommen vorteilhaft in Gestalt ihrer Erdalkali- und insbesondere ihrer Alkalisalze in Frage, z.B. Calciumbromid, Calciumjodid, Magnesiumbromid, Magnesiumjodid, Lithiumbromid, Lithiumjodid und insbesondere Natrium- und Kaliumbromid oder -jodid. Bevorzugte Halogenamide I sind solche, in deren Formel $R^1$ eine Sulfonsäuregruppe, einen Sulfonatrest, insbesondere einen Alkalisulfonatrest wie Natriumsulfonat oder Kaliumsulfonat, eine Sulfonamidgruppe bezeichnet, $R^2$ ein Chloratom, ein Bromatom, einen Alkylrest mit 1 bis 4 Kohlenstoffatomen oder insbesondere ein Wasserstoffatom bedeutet, X ein Bromatom, ein Chloratom oder zweckmäßig ein Wasserstoffatom bezeichnet, $R^1$ und $R^2$ darüber hinaus auch zusammen mit dem benachbarten Stickstoffatom Glieder eines heterocyclischen, 5- oder 6-gliedrigen Ringes, der mindestens eine dem Stickstoffatom benachbarte Sulfongruppe oder Phosphonylgruppe der Formel

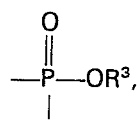

worin $R^3$ für ein Wasserstoffatom oder ein Alkaliatom, insbesondere ein Natriumatom oder Kaliumatom steht, enthält, bezeichnen können oder $R^1$ und $R^2$ zusammen auch den Rest

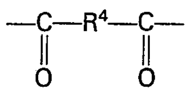

bedeuten können, worin $R^4$ für einen Alkylenrest mit 2 bis 4 Kohlenstoffatomen, den Rest

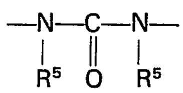

oder den Rest

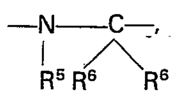

$R^5$ für ein Wasserstoffatom, ein Chloratom oder Bromatom und $R^6$ für einen Alkylrest mit 1 bis 4 Kohlenstoffatomen, insbesondere die Methylgruppe, stehen. An den vorgenannten heterocyclischen Ring kann noch ein Phenylkern anelliert sein. Vorteilhaft enthält der heterocyclische Rest 2 dem Stickstoffatom benachbarte Sulfon- oder Phosphongruppen oder zwei oder drei Sulfonamidogruppen oder Phosphonamidogruppen, insbesondere in demselben Ring, bei mehrkernigen heterocyclischen Resten. Vorgenannte bevorzugte Rest können noch durch unter den Reaktionsbedingungen inerte Gruppen oder Atome, z.B. Chloratome, Bromatome, Alkylgruppen mit 1 bis 4 Kohlenstoffatomen, den Phenylkern substituierende Carboxyl- oder Carboxylatgruppen, substituiert sein.

Als Katalysatoren kommen z.B. in Betracht: Glutarimid, Adipinsäureamid, Succinimid; vorzugsweise Cyanursäure, 5,5-Dimethylhydantoin, Trisulfamid, N-Methyl-sulfaminsäure, Natriumtriimidometaphosphat; entsprechende Gemische vorgenannter Halogenamide I; Sulfaminsäure und ihre Salze, zweckmäßig Alkalisalze wie das Natrium- oder Kaliumsalz, und insbesondere Sulfamid sind besonders bevorzugt, gegebenenfalls im Gemisch mit vorgenannten Halogenamiden I.

Die wäßrigen Hypochloritlösungen der Stufe b) enthalten vorteilhaft von 5 bis 15, insbesondere 12 bis 14 Gewichtsprozent Hypochlorit und keine wesentlichen Mengen, höchstens bis zu 0,01 Mol überschüssiges Alkalihydroxid je Mol Phthalimid/Phthalamidsäure. Bevorzugte Alkalihypochlorite sind das Kaliumsalz oder insbesondere Natriumhypochlorit. Im allgemeinen wird die Reaktion mit einem Verhältnis von Hypohalogenit von 1 bis 2, vorzugsweise 1 bis 1,2 Mol Hypohalogenit je Mol Phthalimid und/oder Phthalamidsäure durchgeführt. Vorteilhaft vermischt man den Ausgangsstoff in seiner wäßrigen, alkalischen Lösung vorgenannter Konzentration aus Stufe a) mit der Alkalihypochloritlösung

in Stufe b) in vorgenanntem Mengenverhältnis in einer Mischungsvorrichtung. Solche Vorrichtungen können Mischzellen, Mischdüsen oder Kammern müt Rührwerken hoher Umdrehungszahl sein. Die Mischung wird in der Regel bei einer Temperatur zwischen 0 und 50°C, vorteilhaft zwischen 25 und 45°C, drucklos oder unter Druck, kontinuierlich durchgeführt.

Die Reaktion wird vorteilhaft in 2 Reaktionsräumen (Stufe c) und d)) unter weitgehender Vermeidung der Rückmischung in beiden Räumen und weitgehend adiabatisch in der ersten Stufe durchgeführt. Die Umsetzung erfolgt in 2 Reaktionsschritten, der Reaktionsstufe c), der Umsetzung des Ausgangsstoffes über das N-chlor-phthalamidsaure Alkalisalz zum phenylisocyanat-2-carbonsauren Alkalisalz und der folgenden Stufe d) der Umsetzung des Alkalisalzes zur Anthranilsäure. Die erste Reaktionsstufe wird weitgehend unter adiabatischen Bedingungen durchgeführt, die entstehende Reaktionswärme erwärmt dabei das Umsetzungsgemisch in der Regel auf eine Temperatur zwischen 20 und 50°C. Aus der Mischungsvorrichtung gelagt das Reaktionsgemisch in den Reaktionsraum der ersten Reaktionsstufe (Stufe c)), der aus einem vorteilhaft engen Reaktionsrohr besteht, und von dort nach der Umsetzung in den Reaktionsraum der folgenden Stufe (Stufe d)). Mischungsvorrichtung, der Reaktionsraum der ersten Stufe und die Lösungen der Ausgangsstoffe brauchen nicht gekühlt zu werden. Ein bevorzugtes Merkmal des Verfahrens nach der Erfindung ist die weitgehende Vermeidung der Rückmischung in Stufe c), ein rascher Entzug des Reaktionsgemisches aus c) und seine Zuführung — unter weitgehender Vermeidung der Rückmischung — in die Stufe d). Zweckmäßig stellt man durch einen engen Querschnitt des Reaktionsrohres der ersten Stufen und Verwendung entsprechender Transportpumpen eine hohe Strömungsgeschwindigkeit des Reaktionsgemisches ein. Als Pumpen können z.B. Strahl-, Rotations-, Kreiskolben-, Wälzkolben-, Schraubenkolben-, Exzenter-, Flügel-, Kreisel-, Axial-, Propeller-pumpen verwendet werden. In einer bevorzugten Ausführungsform des Verfahrens werden die Strömungsgeschwindigkeiten durch Querschnitt und Länge des Reaktionsrohres bestimmt. Vorteilhaft sind z.B. Reaktorquerschnitte von 10 bis 10 000 $mm^2$ und Strömungsgeschwindigkeiten von 0,1 bis 10, insbesondere 0,2 bis 3 m/sec, vorzugsweise 0,5 bis 1 m/sec. Bei diesen Geschwindigkeiten wird in der Regel in einer Verweilzeit von 0,4 bis 40, vorzugsweise von 0,7 bis 20 Sekunden der Ausgangsstoff in der Stufe c) weitgehend über das am Stickstoffatom chlorierte Phthalsäuremonoamid zum Alkalisalz der Phenylisocyanat-2-carbonsäure umgesetzt. Das gebildete Alkalisalz wird, bedingt durch die hohe Strömungsgeschwindigkeit, dem Reaktionsraum der Stufe c) sofort entzogen, der folgenden Stufe zugeführt und dort, im allgemeinen mit einer Verweilzeit von 0,3 bis 150, vorzugsweise von 0,4 bis 40 Sekunden, zur Anthranilsäure umgesetzt. Die hohe Strömungsgeschwindigkeit verhindert gleichzeitig weitgehend eine Rückmischung innerhalb der gesamten Umsetzung des Reaktionsgemisches. Insbesondere wird die Rückmischung des gebildeten Endstoffs mit dem Reaktionsgemisch der Stufe c) vermieden und damit die Bildung von Nebenprodukten durch Umsetzung des Hypohalogenits bzw. des N-chlorierten Phthalsäuremonoamids mit dem Endstoff und/oder durch entsprechende Umsetzungen in den Gemischen der Stufen c) und d) unterdrückt. Die Reaktion wird in Stufe c) bei einer Temperatur von 10 bis 54°C, vorzugsweise zwischen 20 und 54°C, insbesondere zwischen 20 und 45°C, in der Stufe d) von 55 bis 90°C, vorzugsweise von 60 bis 85°C, drucklos oder unter Druck durchgeführt. Am Ende der Reaktionsfolge wird das Reaktionsgemisch entnommen und kann als alkalische Lösung der Anthranilsäure weiterverarbeitet werden, da der Endstoff in ausgezeichneter Reinheit anfällt. Die Isolierung der Endstoffe aus den alkalischen Lösungen kann durch Ausfällen mit Säuren, z.B. Salzsäure oder Schwefelsäure, und nachfolgender Filtration vorgenommen werden.

Die Umsetzung kann vorteilhaft in Stufe c) oder insbesondere Stufe d) unter Zusatz einer großen Zahl von in Wasser und/oder Alkalien löslichen oder mit ihnen mischbaren, in der deutschen Auslegeschrift 2 000 698 beschriebenen Reduktionsmitteln durchgeführt werden. Geeignet sind beispielsweise Hydride wie Natriumborhydrid, Lithiumtriäthoxy-aluminiumhydrid; reduzierende Schwefelverbindungen wie Natriumsulfid, Natriumhydrogensulfid, Ammoniumsulfid, schweflige Säure, Schwefeldioxid, Natriumdithionit, Natriumthiosulfat, Natriumformaldehydsulfoxylat, Thioharnstoffdioxid; Hydrazin und seine Salze, z.B. das Sulfat oder Chlorid; Glucose. Bevorzugte Reduktionsmittel sind Natriumsulfit und Natriumbisulfit. Das Reduktionsmittel kann in stöchiometrischem Verhältnis oder im Überschuß zum zugegebenen Hypohalogenit, vorzugsweise von 0,005 bis 0,1 Äquivalenten Reduktionsmittel je Mol Hypochlorit, verwendet werden. Zweckmäßig kommen Lösungen des Reduktionsmittels in Wasser, z.B. 10- bis 40-gewichtsprozentige, wäßrige Natriumbisulfitlösungen, in Betracht.

Der Zusatz des Reduktionsmittels kann, diskontinuierlich oder in der Regel kontinuierlich, vorteilhaft dem Reaktionsgemisch hinter dem Reaktionsraum der Stufe c) und vor Ende der Stufe d) der Reaktion erfolgen. Man kann das Reduktionsmittel an mehreren Stellen oder zweckmäßig an einer Stelle dem Gemisch während der Umsetzung der in der ersten Reaktionsstufe gebildeten Phenylisocyanat-2-carbonsäure zur Anthranilsäure zusetzen, vorteilhaft direkt nach Beendigung der Umsetzung des Ausgangsstoffes zur Phenylisocyanat-2-carbonsäure. Die Beendigung der Reaktionsstufe c) wird in der Regel durch einen Temperaturanstieg von 20 bis 30°C auf etwa 45 bis 50°C angezeigt. Die Geschwindigkeit des Zusatzes richtet sich in der Regel nach der Strömungsgeschwindigkeit des Reaktionsgemisches, wobei die Konzentration der zugesetzten Lösung und das vorgenannte Verhältnis Reduktionsmittel zu Ausgangshypohalogenit zu berücksichtigen sind. Die Dosierung der Reduktionslösung kann in beliebiger Weise, über Kammern mit Rührwerken, Mischdüsen oder vorzugsweise über

eine Mischzelle, erfolgen. Die Reaktion kann nach Zugabe des Reduktionsmittels im Reaktionsrohr mit hoher Strömungsgeschwindigkeit, z.B. mit 0,2 bis 3 m/sec, oder auch ohne Verminderung der Ausbeute in einem Reaktionsrohr beliebiger Dimensionierung erfolgen. Querschnitt, Strömungsgeschwindigkeit und Temperatur der Ausgangslösungen bestimmen im allgemeinen die Länge der Rohrstrecke, in der die erste Reaktionsstufe c) durchgeführt wird. Beispielsweise ist die Stufe c) bei einem Rohrquerschnitt von 2 200 $mm^2$ und bei einer Strömungsgeschwindigkeit von etwa 1 m/sec bei einer Ausgangstemperatur von etwa 40°C in der Regel nach etwa 1,5 Meter Länge des Reaktionsrohres beendigt, anschließend wird zweckmäßig das Reduktionsmittel zugeführt.

Die nach dem Verfahren der Erfindung herstellbaren Verbindungen sind wertvolle Ausgangsstoffe für die Herstellung von Farbstoffen und Riechstoffen. Bezüglich der Verwendung wird auf die genannten Patentschriften und Ullmanns Encyklopädie der technischen Chemie (4. Auflage), Band 8, Seite 375, verwiesen.

Die in den folgenden Beispielen angeführten Teile bedeuten Gewichtsteile. Sie verhalten sich zu den Volumenteilen wie Kilogramm zu Liter.

Beispiel 1

Man verwendet eine Anlage, die aus einer Mischdüse und einem Reaktionsrohr von 1,1 Meter Länge und 53 Millimeter Innendurchmesser besteht. 590 Teile flüssiges Phthalimid werden stündlich in einer Mischdüse kontinuierlich in 2 103 Teilen wäßriger, 25-gewichtsprozentiger Natronlauge und 4 752 Teilen Wasser gelöst und kontinuierlich 18 Teile/h 30-gewichtsprozentige, wäßrige Lösung des Natriumsalzes der Amidosulfonsäure zugeführt. Die gebildete Lösung wird stündlich in der Mischdüse mit 1 750 Teilen wäßriger Natriumhypochloritlösung (242 Teilen Natriumhypochlorit; 13,8 Gewichtsprozent akt. Chlor) bei 42°C gemischt. Die Strömungsgeschwindigkeit im nachfolgenden Reaktionsrohr beträgt 1,07 Meter pro Sekunde. Die Verweilzeit beträgt eine Sekunde. Das Reaktionsgemisch wird im ersten Teil des Reaktionsrohres (Stufe c)) (0,3 m) weitgehend adiabatisch (von 42 bis 53°C) umgesetzt, wobei im verbleibenden Reaktionsraum eine Selbsterwärmung bis 89°C auftritt. Das Gemisch wird kontinuierlich stündlich mit 25 Teilen 40-gewichtsprozentiger, wäßriger Natriumbisulfitlösung versetzt, auf 10°C abgekühlt und mit Salzsäure auf pH-Wert 4,2 eingestellt, abgesaugt und der Filterrückstand mit Wasser gewaschen und getrocknet. Man erhält stündlich 535 Teile (97% der Theorie) Anthranilsäure (99,3%) vom Fp 146,2°C; Raum-Zeit-Ausbeute: 268 Teile pro Stunde und Liter.

Beispiel 2

Führt man die Umsetzung analog Beispiel 1, aber ohne Zugabe von Amidosulfonsäure durch, so erhält man stündlich 506 Teile (92% der Theorie) Anthranilsäure von einem Gehalt von 99,1 Prozent und Fp 146,1°C. Raum-Zeit-Ausbeute: 27 Teile pro Stunde und Liter.

*Vergleich*

| | Filtrierleistung (Teile Anthranilsäure/h . $m^2$) | Restfeuchte (Gew. % vor dem Trocknen nach dem Filtrieren |
|---|---|---|
| Beispiel 1 | 150 | 6 |
| Beispiel 2 | 130 | 9 |
| Beispiel 1 DAS 1 950 281 | 90 | 20 |
| Beispiel DAS 2 000 698 | 90 | 20 |
| Beispiel 1 DOS 2 328 757 | 120 | 10 |

**Patentansprüche**

1. Verfahren zur kontinuierlichen Herstellung von Anthranilsäure durch Umsetzung von phthalamidsaurem und/oder phthalimidsäurem Alkali mit Hypohalogeniten in wäßrigem Medium, *dadurch gekennzeichnet*, daß man

a) Phthalimid und/oder Phthalamidsäure in wäßriger Alkalilauge mit einem Verhältnis von 3 bis 3,5 Mol Alkalihydroxid je Mol Phthalimid und/oder von 2 bis 2,5-Mol Alkalihydroxid je Mol Phthalamidsäure löst,
b) die so gebildete wäßrige Lösung von phthalamidsaurem und/oder phthalimidsaurem Alkali und eine wäßrige Lösung von Alkalihypochlorit in einer Mischvorrichtung mischt,
c) die erhaltene Mischung im ersten Teil eines Reaktionsrohres mit hoher Strömungsgeschwindigkeit bei 10 bis 54°C unter weitgehend adiabatischen Bedingungen umsetzt, anschließend
d) die aus dem ersten Teil des Reaktionsrohres mit hoher Strömungsgeschwindigkeit abströmende Umsetzungsmischung im zweiten Teil des genannten Rohres bei 55 bis 90°C zu Ende umsetzt und
e) aus der abströmenden alkalischen Umsetzungsmischung in üblicher Weise Anthranilsäure abtrennt.

2. Verfahren nach Anspruch 1,. *dadurch gekennzeichnet,* daß man dem Reaktionsgemisch während der Stufe c) oder d) ein Reduktionsmittel zusetzt.

3. Verfahren nach Anspruch 1 oder 2, *dadurch gekennzeichnet,* daß die Umsetzung in Gegenwart von Brom, Jod und/oder Halogenamiden der Formel

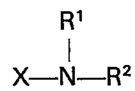 I,

worin $R^1$ eine Sulfonsäuregruppe, einen Sulfonatrest, eine Sulfonamidgruppe bezeichnet, $R^2$ ein Wasserstoffatom, einen aliphatischen Rest, ein Chloratom oder Bromatom bedeutet, X ein Chloratom, Bromatom oder Wasserstoffatom bezeichnet, $R^1$ und $R^2$ darüber hinaus auch zusammen mit dem benachbarten Stickstoffatom Glieder eines heterocylischen Restes, der mindestens eine dem Stickstoffatom benachbarte Sulfongruppe oder Phosphonylgruppe der Formel

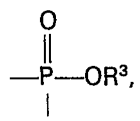

worin $R^3$ für ein Wasserstoffatom oder ein Alkaliatom steht, enthält, bezeichnen können, oder $R^1$ und $R^2$ zusammen auch den Rest

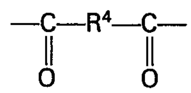

bedeuten können, worin $R^4$ für einen Alkylenrest, den Rest

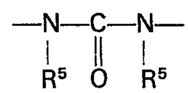

oder den Rest

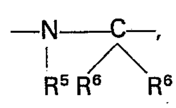

$R^5$ für ein Wasserstoffatom, ein Chloratom oder Bromatom und $R^6$ für einen aliphatischen Rest stehen, durchgeführt wird.

**Revendications**

1. Procédé pour la production en continu d'acide anthranilique par réaction de phtalamidate et/ou de phtalimidate alcalin avec des hypohalogénites en milieu aqueux, caractérisé en ce que:

a) on dissout le phtalimide et/ou l'acide phtalamidique dans une lessive alcaline aqueuse dans un rapport de 3 à 3,5 mol d'hydroxyde alcalin par mol de phtalimide et/ou de 2 à 2,5 mol d'hydroxyde alcalin par mol d'acide phtalamidique;
b) on mélange, dans un dispositif mélangeur, la solution aqueuse ainsi formée de phtalamidate et/ou de phtalimidate alcalin et une solution aqueuse d'hypochlorite alcalin;
c) on fait réagir le mélange obtenu dans la première partie d'un tube de réaction avec une vitesse

d'écoulement élevée, à une température de 10 à 54°C dans des conditions largement adiabatiques; puis

d) on fait réagir jusqu'au bout, à une température de 55 à 90°C dans la seconde partie du tube en question, le mélange réactionnel qui s'écoule de la première partie du tube de réaction à une vitesse d'écoulement élevée, et

e) on sépare l'acide anthranilique du mélange réactionnel alcalin qui s'écoule, en procédant de la manière habituelle.

2. Procédé selon la revendication 1, caractérisé en ce qu'on ajoute un réducteur au mélange réactionnel pendant la phase c) ou d).

3. Procédé selon la revendication 1 ou 2, caractérisé en ce que la réaction est menée en présence de brome, d'iode et/ou d'amides halogénés de formule

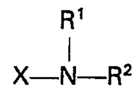

I,

dans laquelle $R^1$ désigne un groupe acide sulfonique, un radical sulfonate, un groupe sulfamide; $R^2$ représente un atome d'hydrogène, un radical aliphatique, un atome de chlore ou un atome de brome; X désigne un atome de chlore, un atome de brome ou un atome d'hydrogène; $R^1$ et $R^2$ peuvent également désigner ensemble, avec l'atome d'azote voisin, des chaînons d'un radical hétérocyclique, qui contient au voisinage de l'atome d'azote au moins un groupe sulfoné ou un groupe phosphonyle de formule

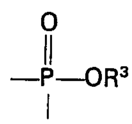

($R^3$ étant mis pour un atome d'hydrogène ou un atome de métal alcalin) ou $R^1$ et $R^2$ peuvent également représenter ensemble le radical

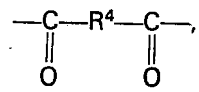

$R^4$ étant mis pour un radical alcoylène, le radical

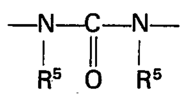

ou le radical

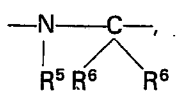

$R^5$ étant un atome d'hydrogène, un atome de chlore ou un atome de brome et $R^6$ étant un radical aliphatique.

**Claims**

1. A process for the continuous preparation of anthranilic acid by reaction of an alkali metal phthalamate and/or alkali metal phthalimidate with a hypohalite in an aqueous medium, wherein

a) phthalimide and/or phthalamic acid is dissolved in an aqueous alkali metal hydroxide solution in a ratio of from 3 to 3.5 moles of alkali metal hydroxide per mole of phthalimide and/or of from 2 to 2.5 moles of alkali metal hydroxide per mole of phthalamic acid,

b) the resulting aqueous solution of alkali metal phthalamate and/or phthalimidate is mixed with an aqueous solution of an alkali metal hypochlorite in a mixing apparatus,

c) the resulting mixture is reacted in the first part of a reaction tube at a high flow rate, at from 10 to 54°C, under substantially adiabatic conditions, thereafter

d) the reaction mixture which leaves the first part of the reaction tube at a high flow rate is allowed to complete the reaction in the second part of the said tube at from 55 to 90°C and

e) anthranilic acid is isolated in the conventional manner from the alkaline reaction mixture which leaves the tube.

2. A process as claimed in claim 1, wherein a reducing agent is added to the reaction mixture during stage c) or d).

3. A process as claimed in claim 1 or 2, wherein the reaction is carried out in the presence of bromine, iodine and/or a haloamide of the formula

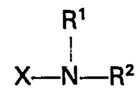

I

where $R^1$ is a sulfonic acid group, a sulfonate radical or a sulfonamide group, $R^2$ is a hydrogen atom, an aliphatic radical, chlorine or bromine, X is chlorine, bromine or hydrogen, and $R^1$ and $R^2$ may also, together with the adjacent nitrogen, be members of a heterocyclic radical which contains at least one sulfone group, or phosphonyl group of the formula

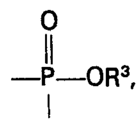

where $R^3$ is hydrogen or an alkali metal, adjacent to the nitrogen, and $R^1$ and $R^2$ may also together be

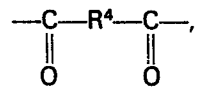

where $R^4$ is alkylene,

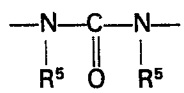

or

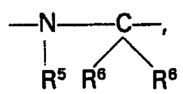

$R^5$ is hydrogen, chlorine or bromine and $R^6$ is an aliphatic radical.